# DEMANDE DE BREVET EUROPEEN

(11) **EP 0 692 238 A1**
(43) Date de publication de la demande: **17.01.1996**
(21) Numéro de dépôt: 95401123.5
(22) Date de dépôt: 15.05.1995
(51) Int. Cl.: A61K 7/00, A61K 7/48, A61K 7/027, A61K 7/32

(54) **Composition cosmétique pour la constitution de bâtons pour les lèvres ou la peau et leurs procédés de préparation**

(30) Priorité: 15.07.1994 FR 9408813
(71) Demandeur: LABORATOIRE DE BIOLOGIE VEGETALE YVES ROCHER, F-56201 La Gacilly (FR)
(72) Inventeur: Deserable, Véronique, F-75017 Paris (FR); Bernard, Jean-Claude, F-94000 Creteil (FR)
(74) Mandataire: Le Guen, Gérard

(57) **Abrégé**

L'invention a pour objet une composition cosmétique pour la constitution de bâtons pour les lèvres ou la peau comprenant une base anhydre dans laquelle est dispersée une émulsion de type eau dans silicone, ladite émulsion étant constituée d'une phase aqueuse dispersée dans une phase lipophile comprenant un ou plusieurs silicones.

## Description

La présente invention concerne une nouvelle composition cosmétique pour la constitution de bâtons pour les lèvres ou la peau, son procédé de préparation ainsi que les bâtons pour les lèvres et la peau proprement dits.

Les bâtons pour les lèvres ou la peau divulgués dans l'art antérieur consistent généralement en un mélange de divers constituants anhydres et/ou lipophiles tels que des cires (naturelles, végétales, minérales ou synthétiques), des huiles (végétales ou minérales) et autres corps gras (esters gras liquides, triglycérides synthétiques et esters gras solides).

Afin de permettre une application uniforme sur la peau, d'éviter toute consistance granuleuse de la formulation et d'assurer sa stabilité au stockage, on a mis au point de nombreuses compositions en déterminant la nature et les proportions respectives des constituants devant être amalgamés.

La préparation de bâtons colorés pour les lèvres est l'une des préoccupations majeures de l'industrie cosmétique. Elle nécessite notamment l'utilisation de matières colorantes.

Jusqu'à présent, l'emploi de colorants hydrosolubles était cependant limité dans la mesure où une mauvaise répartition des colorants hydrosolubles dans la base lipophile était généralement observée, en raison des propriétés hydrophiles de ces constituants.

Pour cette raison les colorants hydrosolubles sont introduits en très faibles quantités dans les compositions de rouge à lèvres en l'état de la technique.

Comme alternative on a préconisé l'utilisation de pigments tels que des oxydes de fer, de chrome ou de manganèse, des bleus outremer, des laques organiques, des colorants naturels ou des nacres. Ceux-ci sont préalablement broyés dans un liant gras avant d'être ajoutés au mélange. L'utilisation de ces pigments pose cependant le problème de leur mauvaise incorporation dans le mélange en raison d'une imprégnation imparfaite de la base lipophile sur les particules, ce qui conduit à un aspect granuleux de la composition résultante. Afin de pallier cet inconvénient on a proposé la mise en oeuvre de procédés complexes, opérant par exemple sous-vide.

De façon générale, les compositions de l'état de la technique ne permettent pas l'incorporation de quantités efficaces de substances hydrosolubles. Or, l'addition de telles substances présente un grand intérêt du point de vue cosmétologique. Comme substances hydrophiles on mentionnera en plus des colorants déjà cités les produits hydratants, les produits adoucissants, les produits émollients, les produits calmants et restructurants, les produits astringents, les produits rafraîchissants, les produits nourrissants et les oligo-éléments.

La présente invention vise à fournir des bâtons pour les lèvres ou la peau pouvant contenir une proportion importante de telles substances hydrophiles.

Les compositions de l'invention sont de plus caractérisées par une texture particulièrement agréable, conférant à la peau un aspect doux, velouté et brillant. Leur étalement sur la peau est aisé et le toucher non gras qu'elles procurent est très appréciable.

Ces propriétés des bâtons pour les lèvres et la peau de l'invention résultent de leur formulation particulière.

L'invention a plus précisément pour premier objet une composition cosmétique pour la constitution de bâtons pour les lèvres ou la peau comprenant une base anhydre dans laquelle est dispersée une émulsion eau dans silicone.

La base anhydre se compose des constituants habituellement utilisés en cosmétique, tels que les cires, les huiles ou corps gras définis ci-dessus. Elle peut comprendre également d'autres additifs tels que des conservateurs, des parfums, voir même des pigments.

L'émulsion eau dans silicone est constituée d'une phase aqueuse dispersée dans une phase lipophile comprenant un ou plusieurs silicones.

Selon l'invention on entend par silicone des polymères organosilylés comprenant des liaisons Si-O-Si. Cette définition comprend un grand nombre de silicones connus dans la technique et commercialisés tels que : les cétyl diméthicones, les cyclométhicones, les diméthicones, les diméthicone copolyols, les acétates de diméthicone copolyols, les éthers d'alkyle en C₁-C₅ et de diméthicone copolyol-PG-bétaines, les silylates de diméthicone, les copolymères de diméthicone/thiosulfate de sodium et de PG-propyldiméthicone, les diméthiconols, les méthicones, les phénéthyl disiloxanes, les stéaroxy diméthicones, les phényl diméthicones, les copolymères diméthycone/stéaroxyméthicone et les stéaroxytriméthylsilanes.

Comme fabricants de silicones on peut mentionner DOW CORNING (USA), GOLDSCHMIDT (ALLEMAGNE), RHONE POULENC (FRANCE), UNION CARBIDE (USA) et BAYER (ALLEMAGNE).

Ces différents silicones ont été désignés d'après la nomenclature du dictionnaire de la C.T.F.A. (Cosmetics, Toiletries and Fragrance Association).

Un groupe préféré de silicones est constitué des polydiméthylsiloxanes, des polydiméthylsiloxanes modifiés par des groupes organiques ioniques et non ioniques et des méthylphénylpolysiloxanes.

Comme polydiméthylsiloxanes on citera particulièrement les diméthicones de formule : 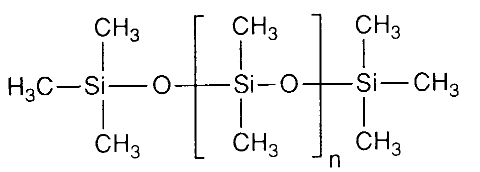
où n est un entier inférieur à 5 000, et les cyclométhicones de formule : 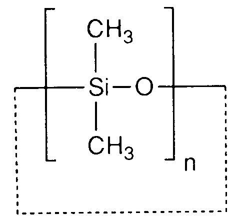
où n est un entier inférieur à 10.

De même parmi les polydiméthylsiloxanes modifiés par des groupements non ioniques, on compte les diméthicone copolyols lesquels sont des polymères de diméthylsiloxane présentant des chaînes latérales de type polyoxyéthylène et/ou polyoxypropylène, les stéaroxy diméthicones lesquels sont des polymères de diméthylpolysiloxane comportant à leurs extrémités des groupes stéaroxy terminaux, et les stéaryl diméthicones.

Enfin, en tant que méthylphénylpolysiloxanes préférés, on mentionnera plus particulièrement les phenyldiméthicones de formule 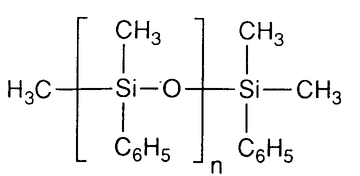
où n est un entier inférieur à 5 000.

Les propriétés physico-chimiques des silicones utilisables selon l'invention et notamment leur hydrophobicité, leur stabilité thermique, leur solubilité dans les milieux organiques, leur viscosité, leur propriétés émulsionnantes, et leur substantivité varient en fonction de leur structure, linéaire, ramifiée ou cyclique, de la nature des groupements fonctionnels qu'ils comportent et de leur masse moléculaire.

A titre d'exemple les diméthicones utilisables selon l'invention ont une viscosité cinématique comprise entre 1.10⁻m /s et 6.10⁻m/s, leur tension superficielle étant d'environ 2.10⁻N/m.

De même les cyclométhicones préférablement utilisés ont une tension superficielle d'environ 17.10⁻³N/m.

On signalera par ailleurs les propriétés émulsionnantes d'un grand nombre de polydiméthylsiloxanes modifiés. Les diméthicones polyols sont connus notamment pour leur propriété émulsionnantes.

C'est notamment en jouant sur ces différences de propriétés des silicones que l'homme du métier pourra modifier les propriétés physico-chimiques de l'émulsion résultante, en sélectionnant les combinaisons appropriées de silicones.

De préférence l'ensemble des silicones contenus dans la composition de l'invention représente de 1 à 50% en poids de l'émulsion, mieux encore de 5 à 30%, ou bien encore de 10 à 25%.

Au côté des constituants silicones, la phase lipophile de l'émulsion peut comprendre un ester tel que l'ester d'un acide et d'un alcool, linéaires ou ramifiés, en C₁-C₃₀.

L'ester contribue à améliorer la cohésion de la composition de l'invention de par son excellente compatibilité avec les silicones de l'émulsion d'une part, et avec les différents constituants de la base anhydre d'autre part.

L'éthyl-2-hexyl laurate est particulièrement indiqué comme ester, dans la mesure où il est très bien toléré par la peau.

En règle générale le rapport massique de l'ester à l'ensemble des silicones est compris entre 1:3 et 2:1.

La phase lipophile peut contenir d'autres types d'additifs, tels que les anti-oxydants, les colorants liposolubles, les parfums, les filtres solaires liposolubles.

L'homme du métier sait qu'il peut utiliser, en tant qu'anti-oxydants, l'acide sorbique, l'acide citrique, le palmitate d'ascorbyle, du butylhydroxyanisol (BHA) et du butylhydroxytoluène (BHT), la vitamine E et ses dérivés.

Toutefois la teneur en additifs de la phase lipophile de l'émulsion ne dépassera pas les 10% en poids, de préférence 5%.

La phase aqueuse des compositions de l'invention permet l'incorporation de nombreuses substances hydrosolubles d'activités variées. Suivant le but recherché, on peut envisager d'ajouter à la phase aqueuse des produits hydratants tels que l'urée, le hyaluronate de sodium, le pyrrolidone carboxylate de sodium (sodium PCA) ou le lactate de sodium, des produits adoucissants tels que D-panthénol, des produits émollients, tels que le polyétylène glycol, des produits humectants, tels que la glycérine, le propylène glycol, le sorbitol ou le butylène glycol, des produits calmants et restructurants tels que l'allantoïne, des produits astringents tels que l'alcloxa, des produits de protection UV tel que l'acide 2-phényl-benzimidazole-5-sulfonique, des produits rafraîchissants tels que le menthol, des produits nourrissants tels que le miel d'acacia ou des vitamines hydrosolubles, des oligo-éléments, ou des colorants hydrosoblubles synthétiques tels que le bleu n° 1, le jaune n° 5, le rouge n° 4, le vert n° 3 ou naturels tels que le rouge de betterave, le caramel, la chlorophylle ou la curcumine.

La teneur en eau de l'émulsion est préférablement comprise entre 30 et 90% en poids, mieux encore entre 50 et 80% en poids.

C'est la dispersion de l'émulsion eau dans silicone dans la base anhydre des compositions de l'invention qui confère à celles-ci leur consistance particulièrement agréable et assure le toucher non gras très recherché en cosmétique.

Dans un mode de réalisation préféré, l'émulsion eau dans silicone utilisée selon l'invention représente 0,5 à 50% en poids de la composition cosmétique de l'invention, mieux encore de 10 à 40% en poids.

Afin de réaliser l'émulsion et d'assurer sa stabilité, il est nécessaire d'y introduire un agent émulsionnant. De tels agents sont connus dans la technique.

Bien que la nature même de l'agent émulsionnant ne soit pas essentielle selon l'invention, il est préférable de choisir un silicone aux propriétés émulsionnantes tels qu'un polydiméthylsiloxane modifié et par exemple un diméthicone copolyol.

Par ailleurs, de façon à contrôler la viscosité et la consistance de l'émulsion résultante eau dans silicone, il peut être utile d'y incorporer un gélifiant.

Les gélifiants utilisables dans l'invention sont ceux généralement utilisés en cosmétique. Comme exemples de tels gélifiants on peut citer les bentones modifiées, les gommes xanthanes, la carboxyméthyl cellulose, les galactomannanes, les acides polyacryliques réticulées, la silice, la gélatine, les alginates et pectines ainsi que le gel d'agar agar.

De nombreux autres types d'ingrédients peuvent être ajoutés à l'émulsion tels que des conservateurs ou des parfums.

L'invention concerne également un procédé de préparation des compositions de l'invention.

Ce procédé comprend les étapes consistant à
a) maintenir sous agitation une phase lipophile comprenant notamment un ou plusieurs silicones à une température comprise entre la température ambiante et 100°C jusqu'à complète homogénéisation du mélange;
b) préparer simultanément une phase aqueuse homogène constituée d'eau éventuellement additionnée d'une ou plusieurs substances hydrosolubles;
c) incorporer sous agitation la phase aqueuse préparée à l'étape b) dans la phase lipophile préparée à l'étape a);
d) le cas échéant refroidir l'émulsion résultante à la température ambiante;
e) mettre en dispersion ladite émulsion dans une base anhydre prévue pour la constitution de bâton pour les lèvres ou la peau.

Afin d'homogénéiser la solution aqueuse de l'émulsion, il est possible de la chauffer en portant par exemple la solution à une température comprise entre la température ambiante et 90°C.

Lorsque des anti-oxydants ou esters sont ajoutés à l'émulsion ceux-ci sont incorporés dans la phase lipophile avant l'étape c). Dans le même temps un émulsionnant est également introduit.

Lorsqu'un gélifiant est utilisé celui-ci peut être soit ajouté à l'émulsion suite à l'incorporation de la phase aqueuse dans la phase lipophile, soit dispersé dans l'une de ces phases ou bien encore dans les deux phases.

Il est par ailleurs souhaitable de n'ajouter les additifs éventuels de l'émulsion tels que conservateurs ou parfums qu'au cours du refroidissement de l'émulsion, c'est-à-dire pendant l'étape d) en raison de leur possible instabilité thermique.

Enfin selon un mode de réalisation préféré les colorants hydrosolubles utilisés sont incorporés à l'émulsion au cours de de l'étape d), éventuellement mélangé à des parfums ou conservateurs, et ceci essentiellement en vue d'éviter leur dégradation à la chaleur.

Les compositions de l'invention peuvent se présenter sous la forme de bâtons pour les lèvres ou la peau.

Comme exemples de bâtons pour les lèvres ou la peau rentrant dans le cadre de l'invention on peut citer les bâtons de rouge à lèvres, les sticks déodorants, les sticks pour les mains, le buste et les pieds.

La mise en forme des bâtons pour les lèvres ou la peau est réalisée de façon conventionnelle.

Le composition de l'invention est coulée à l'état fondu dans des moules prévus à cet effet. Les moules sont remplis à ras bord de façon à éviter la formation d'une dépression au centre des moules. Après refroidissement et démoulage on obtient les bâtons de l'invention.

Les exemples suivants sont donnés uniquement à titre illustratif de la présente invention et ne doivent en aucun cas être considérés comme limitatifs de la portée de celle-ci.

### Exemple 1:

L'exemple 1 illustre plus précisément la préparation de diverses émulsions eau dans silicone.

Dans les formulations proposées, la phase lipophile de l'invention, notée A, contient en plus des silicones, l'éthyl-2-hexyl-laurate, en tant qu'ester, et en tant qu'anti-oxydant, l'acide sorbique.

Dans la phase aqueuse de l'émulsion, notée B, ont été incorporées quelques substances hydrosolubles typiques.

L'utilisation de bentone modifiée, de carboxyméthyl cellulose, de silice et de gélatine en tant que gélifiant (phase C) a par ailleurs été illustrée. Enfin la composition de l'exemple 3 prévoit l'incorporation de colorants hydrosolubles.

Le procédé de préparation comprend les étapes suivantes consistant à :
- chauffer sous-agitation les éléments de A à la température nécessaire pour la complète homogénéisation du mélange;
- préparer une solution aqueuse homogène des constituants de B en la portant si nécessaire à une température comprise entre la température ambiante et 90°C;
- incorporer sous agitation la phase aqueuse B dans la phase lipophile A;
- disperser dans l'émulsion résultante la phase C préalablement homogénéisée ou préalablement dispersée dans une partie de la phase appropriée et comprenant l'ensemble des gélifiants utilisés
- porter le mélange à 25°C
- incorporer alors la phase D, préalablement homogénéisée, comprenant les parfums, conservateurs et le cas échéant les colorants hydrosolubles.

Dans les formulations suivantes les pourcentages indiqués sont des pourcentages en poids.
**Formulation (a)**

| | | |
|---|---|---|
| phase A | Diméthicone copolyol | 4,0 à 6,0 |
| phase A | Acide sorbique | 0,2 à 0,5 |
| phase A | Ethyl-2-hexyl laurate | 5,0 à 15,0 |
| phase A | Diméthicone copolymère | 3,0 à 6,0 |
| phase A | Polysiloxane | 5,0 à 10,0 |
| | | |
| phase B | Eau | QSP 100 |
| phase B | Hyaluronate de sodium | 0,1 à 0,5 |
| phase B | Propylène glycol | 5,0 à 10,0 |
| | | |
| phase C | Bentone modifiée | 1,5 à 3,0 |
| | | |
| phase D | Conversateurs | 0,5 à 1,0 |
| phase D | Parfum | 0,1 à 0,3 |

Le polysiloxane peut être un polydiméthylsiloxane ou un polyphénylméthylsiloxane.
**Formulation (b)** 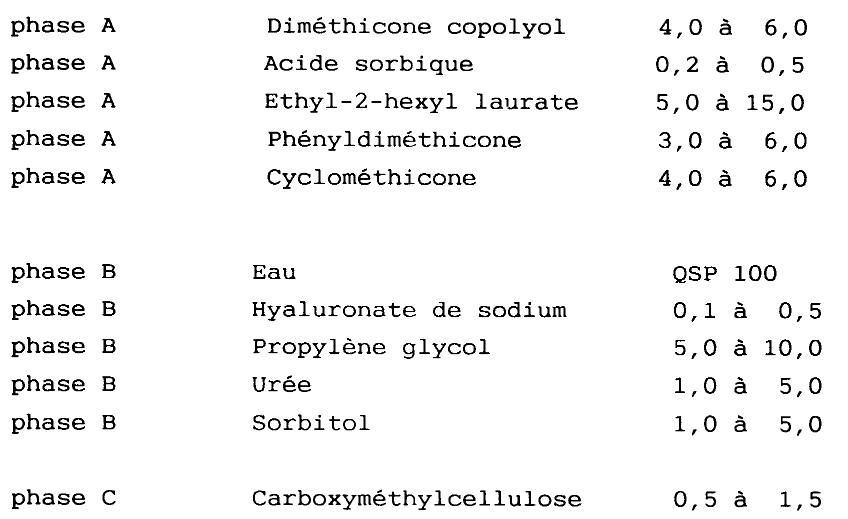 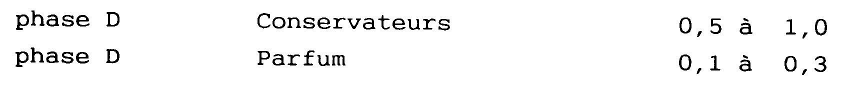
**Formulation (c)**

| | | |
|---|---|---|
| phase A | Diméthicone copolyol | 4,0 à 6,0 |
| phase A | Acid sorbique | 0,2 à 0,5 |
| phase A | Ethyl-2-hexyl laurate | 5,0 à 15,0 |
| phase A | Stéaroxydiméthicone | 1,0 à 2,5 |
| phase A | Diméthylpolysiloxane | 3,0 à 6,0 |
| | | |
| phase B | Eau | QSP 100 |
| phase B | Hyaluronate de sodium | 0,1 à 0,5 |
| phase B | Propylène glycol | 5,0 à 10,0 |
| | | |
| phase C | Gélatine | 0,5 à 2,0 |
| phase C | Silice | 0,4 à 1,0 |
| | | |
| phase D | Colorant hydrosoluble | 0,05 à 0,2 |
| phase D | Conservateurs | 0,5 à 1,0 |
| phase D | Parfum | 0,1 à 0,3 |

On notera que dans chaque cas, la proportion d'eau ajoutée est la quantité d'eau nécessaire pour porter le poids de la composition à 100%

### Exemple 2 :

L'exemple 2 illustre la préparation d'un bâton pour les lèvres ou la peau à base d'une émulsion eau dans silicone.

Les différents constituants du bâton préparé sont les suivants (en % en poids) :

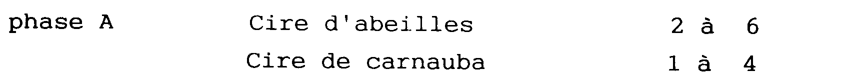

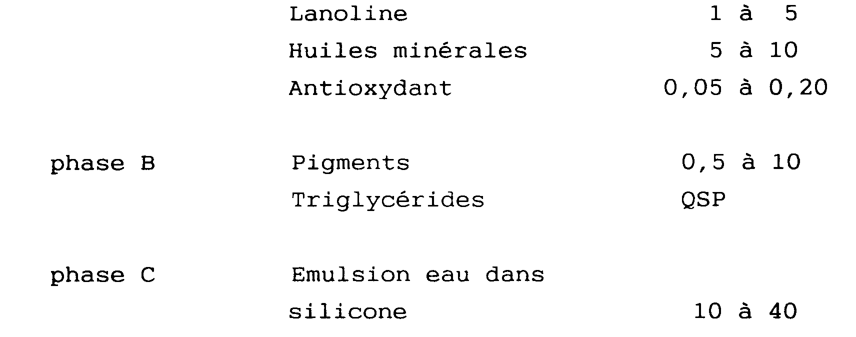

L'émulsion eau dans silicone utilisée peut être l'une quelconque deds émulsions (a), (b) ou (c) décrites à l'exemple 1.

Dans un premier temps les constituants de la phase A sont mélangés à l'état fondu à une température comprise entre 70 et 90°C.

Dans le même temps les pigments sont broyés dans le liant gras de la phase B.

La phase B est ensuite incorporée à la phase A sous agitation. Au mélange maintenu sous agitation est alors ajouté la phase C.

Une fois l'homogénéisation obtenue, la pâte résultante est coulée dans un moule prévu à cet effet. Le démoulage du bâton a lieu à température ambiante.

## Revendications

1. Composition cosmétique pour la constitution de bâtons pour les lèvres ou la peau comprenant une base anhydre dans laquelle est dispersée une émulsion de type eau dans silicone, ladite émulsion étant constituée d'une phase aqueuse dispersée dans une phase lipophile comprenant un ou plusieurs silicones.

2. Composition selon la revendication 1, caractérisée en ce que ledit ou lesdits silicones représentent de 1 à 50% en poids de l'émulsion.

3. Composition selon l'une quelconque des revendications 1 à 2, caractérisée en ce que ledit ou lesdits silicones sont choisis parmi les cétyldiméthicones, les cyclométhicones, les diméthicones, les diméthicone copolyols, les acétates de diméthicone copolyols, les éthers d'alkyle en C₁-C₅ et de diméthicone copolyol-PG-bétaines, les silylates de diméthicone, les copolymères de diméthicone/thiosulfate de sodium et de PG-propyldiméthicone, les diméthiconols, les méthicones, les phénéthyl disiloxanes, les stéaroxy diméthicones, les phényl diméthicones, les copolymères diméthicone/stéaroxyméthicone et les stéaroxytriméthylsilanes.

4. Composition selon l'une quelconque des revendications 1 à 3, caractérisée en ce que ledit ou lesdits silicones sont choisis parmi les polydiméthylsiloxanes, et notamment les diméthicones et cyclométhicones; les polydiméthylsiloxanes modifiés par des groupements organiques ioniques ou non ioniques et notamment les diméthicone copolyols, les stéaryl diméthicones ou les stéaroxy diméthicones; et les méthylphénylpolysiloxanes et notamment les phényldiméthicones.

5. Composition selon l'une quelconque des revendications 1 à 4, caractérisée en ce que la phase lipophile de l'émulsion contient en outre un ester, tel que l'ester d'un acide et d'un alcool, linéaires ou ramifiés, en C₁-C₃₀.

6. Composition selon l'une quelconque des revendications 1 à 5, caractérisée en ce que la phase aqueuse de l'émulsion comprend des substances hydrosolubles telles que des produits hydratants, des produits adoucissants, des produits émollients, des produits humectants, des produits calmants et restructurants, des produits astringents, des produits rafraîchissants, des produits nourrissants, des oligo-éléments, des colorants hydrosolubles ou des filtres solaires hydrosolubles.

7. Composition selon l'une quelconque des revendications 1 à 6, caractérisée en ce que l'émulsion comprend un agent émulsionnant.

8. Composition selon la revendication 7, caractérisée en ce que l'agent émulsionnant est un silicone aux propriétés émulsionnantes et notamment un polydiméthylsiloxane modifié tel qu'un diméthicone copolyol.

9. Composition selon l'une quelconque des revendications 1 à 8, caractérisée en ce que l'émulsion comprend en outre un gélifiant tel qu'une bentone modifiée, une gomme xanthane, de la carboxyméthylcellulose, un galactomannane, un acide polyacrylique réticulé, de la silice, de la gélatine, un alginate, une pectine ou du gel agar-agar.

10. Procédé de préparation d'une composition selon l'une quelconque des revendications 1 à 9, caractérisé en ce qu'il comprend les étapes consistant à :
a) maintenir sous agitation une phase lipophile comprenant notamment un ou plusieurs silicones à une température comprise entre la température ambiante et 100°C jusqu'à complète homogénéisation du mélange;
b) préparer simultanément une phase aqueuse homogène constituée d'eau éventuellement additionnée d'une ou plusieurs substances hydrosolubles;
c) incorporer sous agitation la phase aqueuse préparée à l'étape b) dans la phase lipophile préparée à l'étape a);
d) le cas échéant, refroidir l'émulsion résultante à la température ambiante;
e) mettre en dispersion ladite émulsion dans une base anhydre prévue pour la constitution de bâton pour les lèvres ou la peau.

11. Procédé selon la revendication 10, caractérisé en ce que l'on incorpore un gélifiant dans l'émulsion préparée à l'étape c) ou bien l'on disperse ledit gélifiant dans une partie de la phase aqueuse ou dans une partie de la phase lipophile ou encore dans les deux.

12. Procédé selon l'une quelconque des revendications 10 à 11, caractérisé en ce que lorsque la composition comprend des colorants hydrosolubles, ceux-ci sont ajoutés à l'émulsion au cours de l'étape d).

13. Composition selon l'une quelconque des revendications 1 à 9, sous forme d'un bâton pour les lèvres ou la peau.
